# EUROPÄISCHE PATENTSCHRIFT

(11) **EP 1 888 545 B1**
(45) Veröffentlichungstag und Bekanntmachung des Hinweises auf die Patenterteilung: **25.08.2010**
(21) Anmeldenummer: 06755210.9
(22) Anmeldetag: 16.05.2006
(51) Int. Cl.: C07D 277/20, A01N 43/78

(54) **THIAZOLCARBONSÄUREANILIDE**
THIAZOLE CARBOXYLIC ACID ANILIDES
ANILIDES D'ACIDES THIAZOLCARBOXYLIQUES

(30) Priorität: 18.05.2005 DE 102005023606
(43) Veröffentlichungstag der Anmeldung: 20.02.2008
(73) Patentinhaber: BASF SE, 67056 Ludwigshafen (DE)
(72) Erfinder: DIETZ, Jochen, 68167 Mannheim (DE); GEWEHR, Markus, 56288 Kastellaun (DE); GROTE, Thomas, 67157 Wachenheim (DE); GRAMMENOS, Wassilios, 67071 Ludwigshafen (DE); HÜNGER, Udo, 68167 Mannheim (DE); MÜLLER, Bernd, 67227 Frankenthal (DE); SCHIEWECK, Frank, 67258 Hessheim (DE); SCHWÖGLER, Anja, 68165 Mannheim (DE); LOHMANN, Jan Klaas, 68167 Mannheim (DE); RHEINHEIMER, Joachim, 67063 Ludwigshafen (DE); RENNER, Jens, 68163 Mannheim (DE); SCHÄFER, Peter, 67308 Ottersheim (DE)
(86) Internationale Anmeldenummer: PCT/EP2006/062345
(87) Internationale Veröffentlichungsnummer: WO 2006/122933

(56) Entgegenhaltungen:
- EP-A- 0 589 301
- WO-A-03/066610

## Beschreibung

Die vorliegende Erfindung betrifft Thiazolcarbonsäureanilide der Formel I 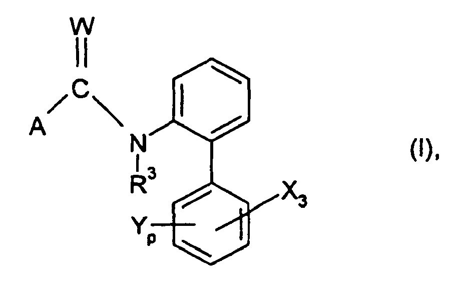 in der die Variablen folgende Bedeutungen haben:
- A: 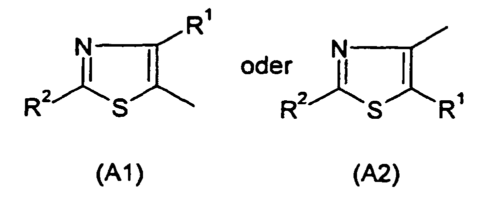
- X: Halogen;
- Y: Cyano, Nitro, C₁-C₄-Alkyl, C₁-C₄-Halogenalkyl, Methoxy oder Methylthio;
- p: 0 oder 1;
- R¹: Wasserstoff, Halogen, C₁-C₄-Nkyl oder C₁-C₄-Halogenalkyl;
- R²: Wasserstoff, Methyl oder Halogen;
- R³: Wasserstoff, Methyl oder Ethyl;
- W: Sauerstoff oder Schwefel.

Dabei können die Substituenten X unabhängig voneinander unterschiedliche Bedeutungen haben.

Außerdem betrifft die Erfindung Verfahren zur Herstellung dieser Verbindungen, sie enthaltende Mittel und Verfahren zu deren Verwendung zur Bekämpfung von Schadpilzen.

Aus der Literatur sind Thiazolcarbonsäureanilide mit fungizider Wirkung bekannt. So werden beispielsweise in der EP-A 545 099 und EP-A 589 301 Biphenylanilide diesen Typs beschrieben, die an der Biphenyl-Gruppe eine Monosubstitution aufweisen.

In der WO 03/066609 werden spezifische Trifluormethyl-thiazolyl-carboxanilide beschrieben und deren fungizide Wirkung. Die beschriebenen Verbindungen sind an der Biphenyl-Gruppe zweifach substituiert.

Aus der WO 03/066610 sind spezifische Difluormethyl-thiazolyl-carboxanilide beschrieben, die an der Biphenyl-Gruppe mono- oder disubstituiert sind.

Aufgabe der vorliegenden Erfindung war es, Thiazolcarbonsäureanilide mit verbesserter fungizider Wirkung aufzufinden im Vergleich zu den Verbindungen des Standes der Technik.

Demgemäß wurden die eingangs definierten Verbindungen I gefunden.

Außerdem wurden Verfahren zur Herstellung dieser Verbindungen, sie enthaltende Mittel und Verfahren zu deren Verwendung zur Bekämpfung von Schadpilzen gefunden.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Man erhält die Verbindungen I im allgemeinen dadurch, dass man ein Carbonsäurehalogenid der Formel II in an sich bekannter Weise (z. B. J. March, Advanced Organic Chemistry, 2nd Ed., 382 f, McGraw-Hill, 1977) in Gegenwart einer Base mit einem Anilin der Formel III umsetzt.

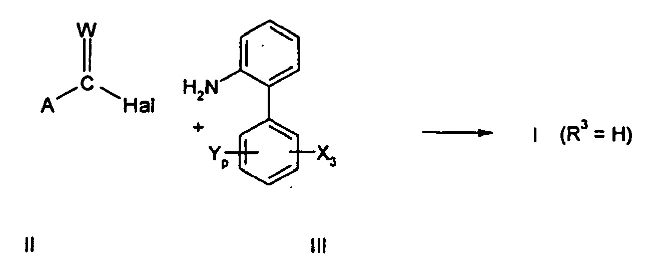

Der Rest Hal in der Formel II steht für ein Halogenatom wie Fluor, Chlor, Brom und Jod, insbesondere für Fluor oder Chlor. Diese Umsetzung erfolgt üblicherweise bei Temperaturen von (-20)°C bis 100°C, vorzugsweise 0°C bis 50°C.

Geeignete Lösungsmittel sind aliphatische Kohlenwasserstoffe wie Pentan. Hexan. Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert-Butanol sowie Methylenchlorid, Dimethylsulfoxid und Dimethylformamid, besonders bevorzugt Toluol, Methylenchlorid und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat und metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z. B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Triethylamin und Pyridin verwendet.

Die Basen werden im allgemeinen in äquimolaren Mengen, bezogen auf die Verbindung II eingesetzt. Sie können aber auch in einem Überschuß von 5 mol-% bis 30 mol-%, vorzugsweise 5 mol-% bis 10 mol%, oder- im Falle der Verwendung von tertiären Aminen - gegebenenfalls als Lösungsmittel verwendet werden.

Die Edukte werden im allgemeinen in etwa äquimolaren Mengen miteinander umgesetzt. Es kann für die Ausbeute vorteilhaft sein, II in einem Überschuß von 1 mol-% bis 20 mol-%, vorzugsweise 1 mol-% bis 10 mol-%, bezogen auf III einzusetzen.

Die für die Herstellung der Verbindungen I benötigten Ausgangsstoffe der Formel II und III sind bekannt oder können analog zu den bekannten Verbindungen synthetisiert werden (Helv. Chim. Acta 60, 978 (1977); Zh. Org. Khim. 26, 1527 (1990); Heterocycles 26, 1885 (1987); Izv. Akad. Nauk. SSSR Ser. Khim., 2160 (1982); THL 28, 593 (1987); THL 29, 5463 (1988)).

Weiterhin wurde gefunden, dass man Verbindungen der Formel 1 dadurch erhält, dass man in bekannter Weise Carbonsäuren der Formel IV mit einem Anilin der Formel III in Gegenwart von Dehydratisierungsmitteln und ggf. einer organischen Base umsetzt.

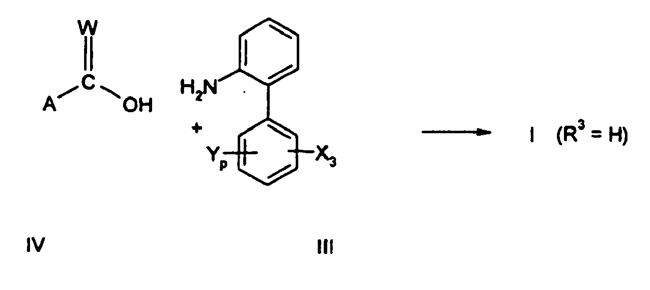

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Methylenchlorid, Toluol und Tetrahydrofuran.

Es können auch Gemische der genannten Lösungsmittel verwendet werden.

Als Dehydratisierungsmittel kommen beispielweise 1,1'-Carbonyldiimidazol, Bis(2-oxo-3-oxazolidinyl)phosphorylchlorid, Carbodiimide wie N,N'-Dicyclohexylcarbodiimid, N-(3-Dimethylaminopropyl)-N'-ethylcarbodiimid, Phosphoniumsalze wie (Benzotriazol-1-yloxy)tris(dimethylamino)phosphoniumhexafluorphosphat, Bromtripyrrolidinophosphoniumhexafluorphosphat, Bromtris(dimethylamino)phosphoniumhexafluorphosphat, Chlortripyn-olidinophosphoniumhexafluorphosphat, Uronium- und Thiuroniumsalze wie O-(Benzotriazol-1-yl)-N,N,N',N'-tetramethyluroniumhexafluorphosphat, O-(7-Azabenzotriazol-1-yl)-N,N,N',N'-tetramethyluroniumhexafluorphosphat, S-(1-Oxido-2-pyridyl)-N,N,N',N'-tetramethylthiuroniumtetrafluorborat, O-(2-Oxo-1(2H)pyridyl)-N,N,N',N'-tetramethyluroniumtetrafluorborat, *O*-[(Ethoxycarbonyl)cyanomethylenamino]-N,N,N',N'-tetramethyluroniumtetrafluorborat, Carbeniumsalze wie (Benzotriazol-1-yloxy)dipyrrolidinocarbeniumhexafluorphosphat, (Benzotriazol-1-yloxy)dipiperidinocarbeniumhexafluorphosphat, O-(3,4-Dihydro-4-oxo-1,2,3-benzotriazin-3-yl)-N,N,N',N'-tetramethyluroniumtetrafluorborat, Chlor-N',N'-bis(tetramethylen)formamidiniumtetrafluorborat, Chlordipyrrolidinocarbeniumhexafluorphosphat, Chlor-N,N,N',N'-bis(pentamethylen)formamidiniumtetrafluorborat, Imidazoliumsalze wie 2-Chlor-1,3-dimethylimidazolidiniumtetrafluorborat, vorzugsweise 1,1'-Carbonyldiimidazol, Bis(2-oxo-3-oxazolidinyl)phosphorylchlorid, N,N'-Dicyclohexylcarbodiimid und N-(3-Dimethylaminopropyl)-N'-ethylcarbodiimid, in Betracht.

Als organische Basen kommen beispielweise tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Pyridin verwendet. Die Basen werden im allgemeinen im Überschuss von 10 mol-% bis 200 mol-%, vorzugsweise von 50 mol-% bis 150 mol-%, bezogen auf die Verbindung IV eingesetzt.

Die Edukte werden im allgemeinen in etwa äquimolaren Mengen miteinander umgesetzt. Es kann für die Ausbeute vorteilhaft sein, eine der Verbindungen in einem Überschuß von 1 mol-% bis 20 mol-%, vorzugsweise 1 mol-% bis 10 mol-%, einzusetzen. Die Dehydratisierungsmittel werden im allgemeinen im Überschuß von 5 mol-% bis 100 mol-%, vorzugsweise 5 mol-% bis 60 mol-%, eingesetzt.

Die für die Herstellung der Verbindungen I benötigten Ausgangsstoffe der Formel III und IV sind bekannt oder können analog zu den bekannten Verbindungen synthetisiert werden.

Die Verbindungen I mit R³ = CH₃ oder C₂H₅ erhält man vorzugsweise dadurch, dass man Verbindungen der Formel I mit R³ = H in bekannter Weise in Gegenwart einer Base mit einem Alkylierungsmittel umsetzt.

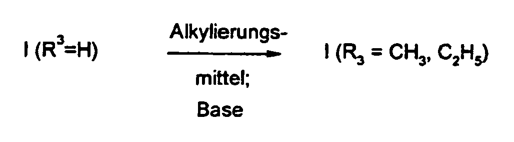

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, sowie Dimethylsulfoxid und Dimethylformamid, besonders bevorzugt Diethylether, tert.-Butylmethylether, Tetrahydrofuran und Dimethylformamid.

Es können auch Gemische der genannten Lösungsmittel verwendet werden.

Geeignete Alkylierungsmittel sind beispielweise Alkylhalogenide wie Methyliodid, Ethyliodid, Methylbromid, Ethylbromid, Methylchlorid und Ethylchlorid, Alkyl(perfluoralkylsulfonate) wie Methyltrifluormethylsulfonat und Ethyltrifluormethylsulfonat, Alkyl(alkylsulfonate) wie Methylmethylsulfonat und Ethylmethylsulfonat, Alkyl(arylsulfonate) wie Methyl-p-tolylsulfonat und Ethyl-p-tolylsulfonat, Oxoniumsalze wie Trimethyloxoniumtetrafluorborat und Triethyloxoniumtetrafluorborat.

Besonders bevorzugt sind Methyliodid, Ethyliodid, Methylbromid, Ethylbromid, Methylchlorid und Ethylchlorid.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Natriumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat und Kalium-tert.-butanolat in Betracht.

Besonders bevorzugt werden Natriumcarbonat, Kaliumcarbonat, Natriumhydrid, Kaliumhydrid, Butyllithium und Kalium-tert.-Butanolat verwendet.

Die Basen werden im allgemeinen in etwa äquimolaren Mengen, bezogen auf die Verbindung I, eingesetzt. Sie können aber auch in einem Überschuß von 5 mol-% bis 30 mol-%, vorzugsweise 5 mol-% bis 10 mol-%, verwendet werden.

Die Edukte werden im allgemeinen in etwa äquimolaren Mengen miteinander umgesetzt. Es kann für die Ausbeute vorteilhaft sein, das Alkylierungsmittel in einem Überschuß von 1 mol-% bis 20 mol-%, vorzugsweise 1 mol-% bis 10 mol-%, bezogen auf I einzusetzen.

Die Verbindungen I, bei denen X für Schwefel steht, sind beispielweise durch Schwefelung der entsprechenden Verbindungen I, bei denen X für Sauerstoff steht, herstellbar (vgl. z.B. D. Petrova & K. Jakobcic, Croat. Chem. Acta 48, 49 (1976) sowie die WO 01/42223).

Im Hinblick auf ihre Verwendung in fungiziden Mitteln kommen Verbindungen der Formel I in Betracht, in der die Substituenten die folgende Bedeutung haben:
Halogen wie Fluor, Chlor, Brom und Jod;
C₁-C₄-Alkyl wie Methyl, Ethyl, n-Propyl, 1-Methylethyl, n-Butyl, 1-Methylpropyl, 2-Methylpropyl und 1,1-Dimethylethyl;
C₁-C₄-Halogenalkyl steht für einen teilweise oder vollständig halogenierten C₁-C₄-Alkylrest, wobei das/die Halogenatom(e) insbesondere Fluor, Chlor und/oder Brom ist/sind, also z.B. Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, Heptafluorpropyl oder Nonafluorbutyl, insbesondere für Halogenmethyl, besonders bevorzugt für CH₂-Cl, CH(Cl)₂, CH₂F, CHF₂, CF₃, CHFCl, CF₂Cl oder CF(Cl)₂.

Im Hinblick auf die biologische Wirkung besonders bevorzugte Verbindungen I sind solche, in denen die Variablen für folgende Reste stechen:
- X: F, Cl, bevorzugt Fluor
- Y: C₁-C₄-Alkyl, C₁-C₄-Halogenalkyl, Methoxy,
bevorzugt Methyl, Difluormethyl, Trifluormethyl, Methoxy;
ganz besonders bevorzugt Methyl, Trifluormethyl;
- p: 0, 1, bevorzugt 0;
- R¹: Wasserstoff, Halogen, C₁-C₄-Alkyl, C₁-C₄-Halogenalkyl;
bevorzugt Wasserstoff, F, Cl, Methyl, Fluormethyl, Difluormethyl, Chlorfluor- methyl, Chlordifluormethyl, Dichlorfluormethyl, Trifluormethyl;
ganz besonders bevorzugt Wasserstoff, Methyl, Fluormethyl, Difluormethyl, Chlorfluormethyl, Trifluormethyl; insbesondere Difluormethyl oder Trifluormethyl;
- R²: Wasserstoff, Halogen, Methyl;
bevorzugt Wasserstoff, F, Cl, Methyl;
ganz besonders bevorzugt Wasserstoff, Cl oder Methyl;
- R³: Wasserstoff oder Methyl; bevorzugt Wasserstoff;
- W: Sauerstoff.

Besonders bevorzugt sind Verbindungen I mit folgenden Substituenten-Kombinationen, in denen die Variablen folgende Bedeutungen haben:
- X: F oder Chlor;
- Y: Methyl, Difluormethyl, Trifluormethyl oder Methoxy;
- P: 0, 1;
- R¹: Wasserstoff, F, Cl, Methyl, Fluormethyl, Difluormethyl, Chlorfluormethyl, Chlordi- fluormethyl, Dichlorfluormethyl, Trifluormethyl;
- R²: Wasserstoff, F, Cl, Methyl;
- R³: Wasserstoff oder Methyl;
- W: Sauerstoff.

Weiterhin bevorzugt sind auch folgende Kombinationen von Variablen mit folgenden Bedeutungen:
- X: F oder Chlor;
- p: Null;
- R¹: Wasserstoff, F, Cl, Methyl, Fluormethyl, Difluormethyl, Chlorfluormethyl, Chlordi- fluormethyl, Dichlorfluormethyl oder Trifluormethyl;
- R²: Wasserstoff, F, Cl oder Methyl; bevorzugt Wasserstoff, Cl oder Methyl;
- R3: Wasserstoff;
- W: Sauerstoff.

Insbesondere sind im Hinblick auf ihre Verwendung als Fungizide die Verbindungen der allgemeinen Formel I-A bevorzugt.

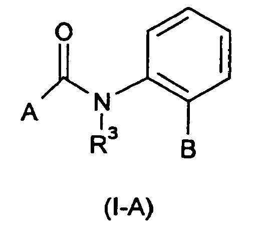

**Tabelle A**

| **Nr.** | **B** | **R¹** |
|---|---|---|
| 1 | 2-Chlor-3,4-difluorphenyl | CF₃ |
| 2 | 2-Chlor-4,5-difluorphenyl | CF₃ |
| 3 | 2-Chlor-5,6-difluorphenyl | CF₃ |
| 4 | 2-Chlor-3,5-difluorphenyl | CF₃ |
| 5 | 2-Chlor-3,6-difluorphenyl | CF₃ |
| 6 | 2-Chlor-4,6-difluorphenyl | CF₃ |
| 7 | 3-Chlor-2,4-difluorphenyl | CF₃ |
| 8 | 3-Chlor-2,5-difluorphenyl | CF₃ |
| 9 | 3-Chlor-2,6-difluorphenyl | CF₃ |
| 10 | 3-Chlor-4,5-difluorphenyl | CF₃ |
| 11 | 3-Chlor-4,6-difluorphenyl | CF₃ |
| 12 | 3-Chlor-5,6-difluorphenyl | CF₃ |
| 13 | 4-Chlor-2,3-difluorphenyl | CF₃ |
| 14 | 4-Chlor-2,5-difluorphenyl | CF₃ |
| 15 | 4-Chlor-2,6-difluorphenyl | CF₃ |
| 16 | 4-Chlor-3,5-difluorphenyl | CF₃ |
| 17 | 2-Fluor-3,4-dichlorphenyl | CF₃ |
| 18 | 2-Fluor-4,5-dichlorphenyl | CF₃ |
| 19 | 2-Fluor-5,6-dichlorphenyl | CF₃ |
| 20 | 2-Fluor-3,5-dichlorphenyl | CF₃ |
| 21 | 2-Fluor-3,6-dichlorphenyl | CF₃ |
| 22 | 2-Fluor-4,6-dichlorphenyl | CF₃ |
| 23 | 3-Fluor-2,4-dichlorphenyl | CF₃ |
| 24 | 3-Fluor-2,5-dichlorphenyl | CF₃ |
| 25 | 3-Fluor-2,6-dichlorphenyl | CF₃ |
| 26 | 3-Fluor-4,5-dichlorphenyl | CF₃ |
| 27 | 3-Fluor-4,6-dichlorphenyl | CF₃ |
| 28 | 3-Fluor-5,6-dichlorphenyl | CF₃ |
| 29 | 4-Fluor-2,3-dichlorphenyl | CF₃ |
| 30 | 4-Fluor-2,5-dichlorphenyl | CF₃ |
| 31 | 4-Fluor-2,6-dichlorphenyl | CF₃ |
| 32 | 4-Fluor-3,5-dichlorphenyl | CF₃ |
| 33 | 2,3,4-Trichlorphenyl | CHF₂ |
| 34 | 2,3,5-Trichlorphenyl | CHF₂ |
| 35 | 2,3,6-Trichlorphenyl | CHF₂ |
| 36 | 2,4,5-Trichlorphenyl | CHF₂ |
| 37 | 2,4,6-Trichlorphenyl | CHF₂ |
| 38 | 3,4,5-Trichlorphenyl | CHF₂ |
| 39 | 2,3,4-Trifluorphenyl | CHF₂ |
| 40 | 2,3,5-Trifluorphenyl | CHF₂ |
| 41 | 2,3,6-Trifluorphenyl | CHF₂ |
| 42 | 2,4,5-Trifluorphenyl | CHF₂ |
| 43 | 2,4,6-Trifluorphenyl | CHF₂ |
| 44 | 3,4,5-Trifluorphenyl | CHF₂ |
| 45 | 2-Chlor-3,4-difluorphenyl | CHF₂ |
| 46 | 2-Chlor-4,5-difluorphenyl | CHF₂ |
| 47 | 2-Chlor-5,6-difluorphenyl | CHF₂ |
| 48 | 2-Chlor-3,5-difluorphenyl | CHF₂ |
| 49 | 2-Chlor-3,6-difluorphenyl | CHF₂ |
| 50 | 2-Chlor-4,6-difluorphenyl | CHF₂ |
| 51 | 3-Chlor-2,4-difluorphenyl | CHF₂ |
| 52 | 3-Chlor-2,5-difluorphenyl | CHF₂ |
| 53 | 3-Chlor-2,6-difluorphenyl | CHF₂ |
| 54 | 3-Chlor-4,5-difluorphenyl | CHF₂ |
| 55 | 3-Chlor-4,6-difluorphenyl | CHF₂ |
| 56 | 3-Chlor-5,6-difiuorphenyl | CHF₂ |
| 57 | 4-Chlor-2,3-difluorphenyl | CHF₂ |
| 58 | 4-Chlor-2,5-difluorphenyl | CHF₂ |
| 59 | 4-Chlor-2,6-difluorphenyl | CHF₂ |
| 60 | 4-Chlor-3,5-difluorphenyl | CHF₂ |
| 61 | 2-Fluor-3,4-dichlorphenyl | CHF₂ |
| 62 | 2-Fluor-4,5-dichlorphenyl | CHF₂ |
| 63 | 2-Fluor-5,6-dichlorphenyl | CHF₂ |
| 64 | 2-Fluor-3,5-dichlorphenyl | CHF₂ |
| 65 | 2-Fluor-3,6-dichlorphenyl | CHF₂ |
| 66 | 2-Fluor-4,6-dichlorphenyl | CHF₂ |
| 67 | 3-Fluor-2,4-dichlorphenyl | CHF₂ |
| 68 | 3-Fluor-2,5-dichlorphenyl | CHF₂ |
| 69 | 3-Fluor-2,6-dichlorphenyl | CHF₂ |
| 70 | 3-Fluor-4,5-dichlorphenyl | CHF₂ |
| 71 | 3-Fluor-4,6-dichlorphenyl | CHF₂ |
| 72 | 3-Fluor-5,6-dichlorphenyl | CHF₂ |
| 73 | 4-Fluor-2,3-dichlorphenyl | CHF₂ |
| 74 | 4-Fluor-2,5-dichlorphenyl | CHF₂ |
| 75 | 4-Fluor-2,6-dichlorphenyl | CHF₂ |
| 76 | 4-Fluor-3,5-dichlorphenyl | CHF₂ |
| 77 | 2,3,4-Trichlorphenyl | CH₂F |
| 78 | 2,3,5-Trichlorphenyl | CH₂F |
| 79 | 2,3,6-Trichlorphenyl | CH₂F |
| 80 | 2,4,5-Trichlorphenyl | CH₂F |
| 81 | 2,4,6-Trichlorphenyl | CH₂F |
| 82 | 3,4,5-Trichlorphenyl | CH₂F |
| 83 | 2,3,4-Trifluorphenyl | CH₂F |
| 84 | 2,3,5-Trifluorphenyl | CH₂F |
| 85 | 2,3,6-Trifluorphenyl | CH₂F |
| 86 | 2.4.5-Trifluorphenyl | CH₂F |
| 87 | 2,4,6-Trifluorphenyl | CH₂F |
| 88 | 3,4,5-Trifluorphenyl | CH₂F |
| 89 | 2-Chlor-3,4-difluorphenyl | CH₂F |
| 90 | 2-Chlor-4,5-difluorphenyl | CH₂F |
| 91 | 2-Chlor-5,6-difluorphenyl | CH₂F |
| 92 | 2-Chlor-3,5-difluorphenyl | CH₂F |
| 93 | 2-Chlor-3,6-difluorphenyl | CH₂F |
| 94 | 2-Chlor-4,6-difluorphenyl | CH₂F |
| 95 | 3-Chlor-2,4-difluorphenyl | CH₂F |
| 96 | 3-Chlor-2,5-difluorphenyl | CH₂F |
| 97 | 3-Chlor-2,6-difluorphenyl | CH₂F |
| 98 | 3-Chlor-4,5-difluorphenyl | CH₂F |
| 99 | 3-Chlor-4,6-difluorphenyl | CH₂F |
| 100 | 3-Chlor-5,6-difluorphenyl | CH₂F |
| 101 | 4-Chlor-2,3-difluorphenyl | CH₂F |
| 102 | 4-Chlor-2,5-difluorphenyl | CH₂F |
| 103 | 4-Chlor-2,6-difluorphenyl | CH₂F |
| 104 | 4-Chlor-3,5-difluorphenyl | CH₂F |
| 105 | 2-Fluor-3,4-dichlorphenyl | CH₂F |
| 106 | 2-Fluor-4,5-dichlorphenyl | CH₂F |
| 107 | 2-Fluor-5,6-dichlorphenyl | CH₂F |
| 108 | 2-Fluor-3,5-dichlorphenyl | CH₂F |
| 109 | 2-Fluor-3,6-dichlorphenyl | CH₂F |
| 110 | 2-Fluor-4,6-dichlorphenyl | CH₂F |
| 111 | 3-Fluor-2,4-dichlorphenyl | CH₂F |
| 112 | 3-Fluor-2,5-dichlorphenyl | CH₂F |
| 113 | 3-Fluor-2,6-dichlorphenyl | CH₂F |
| 114 | 3-Fluor-4,5-dichlorphenyl | CH₂F |
| 115 | 3-Fluor-4,6-dichlorphenyl | CH₂F |
| 116 | 3-Fluor-5,6-dichlorphenyl | CH₂F |
| 117 | 4-Fluor-2,3-dichlorphenyl | CH₂F |
| 118 | 4-Fluor 2,5-dichlorphenyl | CH₂F |
| 119 | 4-Fluor-2,6-dichlorphenyl | CH₂F |
| 120 | 4-Fluor-3,5-dichlorphenyl | CH₂F |
| 121 | 2,3,4-Trichlorphenyl | CHFCl |
| 122 | 2,3,5-Trichlorphenyl | CHFCl |
| 123 | 2,3,6-Trichlorphenyl | CHFCl |
| 124 | 2,4,5-Trichlorphenyl | CHFCl |
| 125 | 2,4,6-Trichlorphenyl | CHFCl |
| 126 | 3,4,5-Trichlorphenyl | CHFCl |
| 127 | 2,3,4-Trifluorphenyl | CHFCl |
| 128 | 2,3,5-Trifluorphenyl | CHFCl |
| 129 | 2,3,6-Trifluorphenyl | CHFCl |
| 130 | 2,4,5-Trifluorphenyl | CHFCl |
| 131 | 2,4,6-Trifluorphenyl | CHFCl |
| 132 | 3,4,5-Trifluorphenyl | CHFCl |
| 133 | 2-Chlor-3,4-difluorphenyl | CHFCl |
| 134 | 2-Chlor-4,5-difluorphenyl | CHFCl |
| 135 | 2-Chlor-5,6-difluorphenyl | CHFCl |
| 136 | 2-Chlor-3,5-difluorphenyl | CHFCl |
| 137 | 2-Chlor-3,6-difluorphenyl | CHFCl |
| 138 | 2-Chlor-4,6-difluorphenyl | CHFCl |
| 139 | 3-Chlor-2,4-difluorphenyl | CHFCl |
| 140 | 3-Chlor-2,5-difluorphenyl | CHFCl |
| 141 | 3-Chlor-2,6-difluorphenyl | CHFCl |
| 142 | 3-Chlor-4,5-difluorphenyl | CHFCl |
| 143 | 3-Chlor-4,6-difluorphenyl | CHFCl |
| 144 | 3-Chlor-5,6-difluorphenyl | CHFCl |
| 145 | 4-Chlor-2,3-difluorphenyl | CHFCl |
| 146 | 4-Chlor-2,5-difluorphenyl | CHFCl |
| 147 | 4-Chlor-2,6-difluorphenyl | CHFCl |
| 148 | 4-Chlor-3,5-difluorphenyl | CHFCl |
| 149 | 2-Fluor-3,4-dichlorphenyl | CHFCl |
| 150 | 2-Fluor-4,5-dichlorphenyl | CHFCl |
| 151 | 2-Fluor-5,6-dichlorphenyl | CHFCl |
| 152 | 2-Fluor-3,5-dichlorphenyl | CHFCl |
| 153 | 2-Fluor-3,6-dichlorphenyl | CHFCl |
| 154 | 2-Fluor-4,6-dichlorphenyl | CHFCl |
| 155 | 3-Fluor-2,4-dichlorphenyl | CHFCl |
| 156 | 3-Fluor-2,5-dichlorphenyl | CHFCl |
| 157 | 3-Fluor-2,6-dichlorphenyl | CHFCl |
| 158 | 3-Fluor-4,5-dichlorphenyl | CHFCl |
| 159 | 3-Fluor-4,6-dichlorphenyl | CHFCl |
| 160 | 3-Fluor-5,6-dichlorphenyl | CHFCl |
| 161 | 4-Fluor-2,3-dichlorrphenyl | CHFCl |
| 162 | 4-Fluor-2,5-dichlorphenyl | CHFCl |
| 163 | 4-Fluor-2,6-dichlorphenyl | CHFCl |
| 164 | 4-Fluor-3,5-dichlorphenyl | CHFCl |
| 165 | 2,3,4-Trichlorphenyl | CF₂Cl |
| 166 | 2,3,5-Trichlorphenyl | CF₂Cl |
| 167 | 2,3,6-Trichlorphenyl | CF₂Cl |
| 168 | 2,4,5-Trichlorphenyl | CF₂Cl |
| 169 | 2,4,6-Trichlorphenyl | CF₂Cl |
| 170 | 3,4,5-Trichlorphenyl | CF₂Cl |
| 171 | 2,3,4-Trifluorphenyl | CF₂Cl |
| 172 | 2,3,5-Trifluorphenyl | CF₂Cl |
| 173 | 2, 3,6-Trifluorphenyl | CF₂Cl |
| 174 | 2,4,5-Trifluorphenyl | CF₂Cl |
| 175 | 2,4,6-Trifluorphenyl | CF₂Cl |
| 176 | 3,4,5-Trifluorphenyl | CF₂Cl |
| 177 | 2-Chlor-3,4-difluorphenyl | CF₂Cl |
| 178 | 2-Chlor-4,5-difluorphenyl | CF₂Cl |
| 179 | 2-Chlor-5,6-difluorphenyl | CF₂Cl |
| 180 | 2-Chlor-3,5-difluorphenyl | CF₂Cl |
| 181 | 2-Chlor-3,6-difluorphenyl | CF₂Cl |
| 182 | 2-Chlor-4,6-difluorphenyl | CF₂Cl |
| 183 | 3-Chlor-2,4-difluorphenyl | CF₂Cl |
| 184 | 3-Chlor-2,5-difluorphenyl | CF₂Cl |
| 185 | 3-Chlor-2,6-difluorphenyl | CF₂Cl |
| 186 | 3-Chlor-4,5-difluorphenyl | CF₂Cl |
| 187 | 3-Chlor-4,6-difluorphenyl | CF₂Cl |
| 188 | 3-Chlor-5,6-difluorphenyl | CF₂Cl |
| 189 | 4-Chlor-2,3-difluorphenyl | CF₂Cl |
| 190 | 4-Chlor-2,5-difluorphenyl | CF₂Cl |
| 191 | 4-Chlor-2,6-difluorphenyl | CF₂Cl |
| 192 | 4-chlor-3,5-difluorphenyl | CF₂Cl |
| 193 | 2-Fluor-3,4-dichlorphenyl | CF₂Cl |
| 194 | 2-Fluor-4,5-dichlorphenyl | CF₂Cl |
| 195 | 2-Fluor-5,6-dichlorphenyl | CF₂Cl |
| 196 | 2-Fluor-3,5-dichlorphenyl | CF₂Cl |
| 197 | 2-Fluor-3,6-dichlorphenyl | CF₂Cl |
| 198 | 2-Fluor-4,6-dichlorphenyl | CF₂Cl |
| 199 | 3-Fluor-2,4-dichlorphenyl | CF₂Cl |
| 200 | 3-Fluor-2,5-dichlorphenyl | CF₂Cl |
| 201 | 3-Fluor-2,6-dichlorphenyl | CF₂Cl |
| 202 | 3-Fluor-4,5-dichlorphenyl | CF₂Cl |
| 203 | 3-Fluor-4,6-dichlorphenyl | CF₂Cl |
| 204 | 3-Fluor-5,6-dichlorphenyl | CF₂Cl |
| 205 | 4-Fluor-2,3-dichlorphenyl | CF₂Cl |
| 206 | 4-Fluor-2,5-dichlorphenyl | CF₂Cl |
| 207 | 4-Fluor-2,6-dichlorphenyl | CF₂Cl |
| 208 | 4-Fluor-3,5-dichlorphenyl | CF₂Cl |
| 209 | 2,3,4-Trichlorphenyl | CFCl₂ |
| 210 | 2,3,5-Trichlorphenyl | CFCl₂ |
| 211 | 2,3,6-Trichlorphenyl | CFCl₂ |
| 212 | 2,4,5-Trichlorphenyl | CFCl₂ |
| 213 | 2,4,6-Trichlorphenyl | CFCl₂ |
| 214 | 3,4,5-Trichlorphenyl | CFCl₂ |
| 215 | 2,3,4-Trifluorphenyl | CFCl₂ |
| 216 | 2.3,5-Trifluorphenyl | CFCl₂ |
| 217 | 2,3,6-Trifluorphenyl | CFCl₂ |
| 218 | 2,4,5-Trifluorphenyl | CFCl₂ |
| 219 | 2,4,6-Trifluorphenyl | CFCl₂ |
| 220 | 3,4,5-Trifluorphenyl | CFCl₂ |
| 221 | 2-Chlor-3,4-difluorphenyl | CFCl₂ |
| 222 | 2-Chlor-4,5-difluorphenyl | CFCl₂ |
| 223 | 2-Chlor-5,6-difluorphenyl | CFCl₂ |
| 224 | 2-Chlor-3,5-difluorphenyl | CFCl₂ |
| 225 | 2-Chlor-3,6-difluorphenyl | CFCl₂ |
| 226 | 2-chlor-4,6-difluorphenyl | CFCl₂ |
| 227 | 3-Chlor-2,4-difluorphenyl | CFCl₂ |
| 228 | 3-Chlor-2,5-difluorphenyl | CFCl₂ |
| 229 | 3-chlor-2,6-difluorphenyl | CFCl₂ |
| 230 | 3-Chlor-4,5-difluorphenyl | CFCl₂ |
| 231 | 3-Chlor-4,6-difluorphenyl | CFCl₂ |
| 232 | 3-Chlor-5,6-difluorphenyl | CFCl₂ |
| 233 | 4-Chlor-2,3-difluorphenyl | CFCl₂ |
| 234 | 4-Chlor-2,5-difluorphenyl | CFCl₂ |
| 235 | 4-Chlor-2,6-difluorphenyl | CFCl₂ |
| 236 | 4-Chlor-3,5-difluorphenyl | CFCl₂ |
| 237 | 2-Fluor-3,4-dichlorphenyl | CFCl₂ |
| 238 | 2-Fluor-4,5-dichlorphenyl | CFCl₂ |
| 239 | 2-Fluor-5,6-dichlorphenyl | CHFCl₂ |
| 240 | 2-Fluor-3,5-dichlorphenyl | CFCl₂ |
| 241 | 2-Fluor-3,6-dichlorphenyl | CFCl₂ |
| 242 | 2-Fluor-4,6-dichlorphenyl | CFCl₂ |
| 243 | 3-Fluor-2,4-dichlorphenyl | CFCl₂ |
| 244 | 3-Fluor-2,5-dichlorphenyl | CHFCl₂ |
| 245 | 3-Fluor-2,6-dichlorphenyl | CFCl₂ |
| 246 | 3-Fluor-4,5-dichlorphenyl | CHFCl₂ |
| 247 | 3-Fluor-4,6-dichlorphenyl | CFCl₂ |
| 248 | 3-Fluor-5,6-dichlorphenyl | CFCl₂ |
| 249 | 4-Fluor-2,3-dichlorphenyl | CFCl₂ |
| 250 | 4-Fluor-2,5-dichlorphenyl | CFCl₂ |
| 251 | 4-Fluor-2,6-dichlorphenyl | CFCl₂ |
| 252 | 4-Fluor-3,5-dichlorphenyl | CFCl₂ |
| 253 | 2,3,4-Trichlorphenyl | CH₃ |
| 254 | 2,3,5-Trichlorphenyl | CH₃ |
| 255 | 2,3,6-Trichlorphenyl | CH₃ |
| 256 | 2.4,5- Trichlorphenyl | CH₃ |
| 257 | 2,4,6-Trichlorphenyl | CH₃ |
| 258 | 3,4,5-Trichlorphenyl | CH₃ |
| 259 | 2,3,4-Trifluorphenyl | CH₃ |
| 260 | 2,3,5-Trifluorphenyl | CH₃ |
| 261 | 2,3,6-Trifluorphenyl | CH₃ |
| 262 | 2,4,5-Trifluorphenyl | CH₃ |
| 263 | 2,4,6-Trtfluorphenyl | CH₃ |
| 264 | 3,4,5-Trifluorphenyl | CH₃ |
| 265 | 2-Chlor-3,4-difluorphenyl | CH₃ |
| 266 | 2-Chlor-4,5-difluorphenyl | CH₃ |
| 267 | 2-Chlor-5,6-difluorphenyl | CH₃ |
| 268 | 2-Chlor-3,5-difluorphenyt | CH₃ |
| 269 | 2-Chlor-3,6-difluorphenyl | CH₃ |
| 270 | 2-Chlor-4,6-difluorphenyl | CH₃ |
| 271 | 3-Chlor-2,4-difluorphenyl | CH₃ |
| 272 | 3-Chlor-2,5-difluorphenyl | CH₃ |
| 273 | 3-Chlor-2,6-difluorphenyl | CH₃ |
| 274 | 3-Chlor-4,5-difluorphenyl | CH₃ |
| 275 | 3-Chlor-4,6-difluorphenyl | CH₃ |
| 276 | 3-Chlor-5,6-difluorphenyl | CH₃ |
| 277 | 4-Chlor-2,3-difluorphenyl | CH₃ |
| 278 | 4-Chlor-2,5-difluorphenyl | CH₃ |
| 279 | 4-Chlor-2,6-difluorphenyl | CH₃ |
| 280 | 4-Chlor-3,5-difluorphenyl | CH₃ |
| 281 | 2-Fluor-3,4-dichlorphenyl | CH₃ |
| 282 | 2-Fluor-4,5-dichlorphenyl | CH₃ |
| 283 | 2-Fluor-5,6-dichlorphenyl | CH₃ |
| 284 | 2-Fluor-3,5-dichlorphenyl | CH₃ |
| 285 | 2-Fluor-3,6-dichlorphenyl | CH₃ |
| 286 | 2-Fluor-4,6-dichlorphenyl | CH₃ |
| 287 | 3-Fluor-2,4-dichlorphenyl | CH₃ |
| 288 | 3-Fluor-2,5-dichlorphenyl | CH₃ |
| 289 | 3-Fluor-2,6-dichlorphenyl | CH₃ |
| 290 | 3-Fluor-4,5-dichlorphenyl | CH₃ |
| 291 | 3-Fluor-4,6-dichlorphenyl | CH₃ |
| 292 | 3-Fluor-5,6-dichlorphenyl | CH₃ |
| 293 | 4-Fluor-2,3-dichlorphenyl | CH₃ |
| 294 | 4-Fluor-2,5-dichlorphenyl | CH₃ |
| 295 | 4-Fluor-2,6-dichlorphenyl | CH₃ |
| 296 | 4-Fluor-3.5-dichlorphenyl | CH₃ |

### Tabelle 1:

Verbindungen der allgemeinen Formel I-A, worin A für A1, R², R³ für Wasserstoff stehen und R¹ und B für jede einzelne Verbindung jeweils einer Zeile der Tabelle A entsprechen.

### Tabelle 2:

Verbindungen der allgemeinen Formel 1-A, worin A für A1, R² für Methyl, R³ für Wasserstoff stehen und R¹ und B für jede einzelne Verbindung jeweils einer Zeile der Tabelle A entsprechen.

### Tabelle 3:

Verbindungen der allgemeinen Formel I-A, worin A für A1, R² für Cl, R³ für Wasserstoff stehen und R¹ und B für jede einzelne Verbindung jeweils einer Zeile der Tabelle A entsprechen.

### Tabelle 4:

Verbindungen der allgemeinen Formel I-A worin A für A1, R² für F, R³ für Wasserstoff stehen und R¹ und B für jede einzelne Verbindung jeweils einer Zeile der Tabelle A entsprechen.

### Tabelle 5:

Verbindungen der allgemeinen Formel I-A, worin A für A1, R² für Wasserstoff, R³ für Methyl stehen und R¹ und B für jede einzelne Verbindung jeweils einer Zeile der Tabelle A entsprechen.

### Tabelle 6:

Verbindungen der allgemeinen Formel I-A, worin A für A1, R², R³ für Methyl stehen und R¹ und B für jede einzelne Verbindung jeweils einer Zeile der Tabelle A entsprechen.

### Tabelle 7:

Verbindungen der allgemeinen Formel I-A, worin A für A1, R² für Cl, R³ für Methyl stehen und R¹ und B für jede einzelne Verbindung jeweils einer Zeile der Tabelle A entsprechen.

### Tabelle 8:

Verbindungen der allgemeinen Formel I-A, worin A für A1, R² für F, R³ für Methyl stehen und R¹ und B für jede einzelne Verbindung jeweils einer Zeile der Tabelle A entsprechen.

### Tabelle 9:

Verbindungen der allgemeinen Formel I-A, worin A für A1, R² für Wasserstoff, R³ für Ethyl stehen und R¹ und B für jede einzelne Verbindung jeweils einer Zeile der Tabelle A entsprechen.

### Tabelle 10:

Verbindungen der allgemeinen Formel I-A, worin A für A1, R² für Methyl, R³ für Ethyl stehen und R¹ und B für jede einzelne Verbindung jeweils einer Zeile der Tabelle A entsprechen.

### Tabelle 11:

Verbindungen der allgemeinen Formel I-A, worin A für A1, R² für Cl, R³ für Ethyl stehen und R¹ und B für jede einzelne Verbindung jeweils einer Zeile der Tabelle A entsprechen.

### Tabelle 12:

Verbindungen der allgemeinen Formel I-A, worin A für A1, R² für F, R³ für Ethyl stehen und R¹ und B für jede einzelne Verbindung jeweils einer Zeile der Tabelle A entsprechen.

### Tabelle 13:

Verbindungen der allgemeinen Formel I-A, worin A für A2, R², R³ für Wasserstoff stehen und R¹ und B für jede einzelne Verbindung jeweils einer Zeile der Tabelle A entsprechen.

### Tabelle 14:

Verbindungen der allgemeinen Formel I-A, worin A für A2, R² für Methyl, R³ für Wasserstoff stehen und R¹ und B für jede einzelne Verbindung jeweils einer Zeile der Tabelle A entsprechen.

### Tabelle 15:

Verbindungen der allgemeinen Formel I-A, worin A für A2, R² für Cl, R³ für Wasserstoff stehen und R¹ und B für jede einzelne Verbindung jeweils einer Zeile der Tabelle A entsprechen.

### Tabelle 16:

Verbindungen der allgemeinen Formel I-A, worin A für A2, R² für F, R³ für Wasserstoff stehen und R¹ und B für jede einzelne Verbindung jeweils einer Zeile der Tabelle A entsprechen.

### Tabelle 17:

Verbindungen der allgemeinen Formel I-A, worin A für A2, R² für Wasserstoff, R³ für Methyl stehen und R¹ und B für jede einzelne Verbindung jeweils einer Zelle der Tabelle A entsprechen.

### Tabelle 18:

Verbindungen der allgemeinen Formel I-A, worin A für A2, R², R³ für Methyl stehen und R¹ und B für jede einzelne Verbindung jeweils einer Zeile der Tabelle A entsprechen.

### Tabelle 19:

Verbindungen der allgemeinen Formel I-A, worin A für A2, R² für Cl, R³ für Methyl stehen und R¹ und B für jede einzelne Verbindung jeweils einer Zeile der Tabelle A entsprechen.

### Tabelle 20:

Verbindungen der allgemeinen Formel I-A. worin A für A2, R² für F, R³ für Methyl stehen und R¹ und B für jede einzelne Verbindung jeweils einer Zeile der Tabelle A entsprechen.

### Tabelle 21:

Verbindungen der allgemeinen Formel I-A, worin A für A2, R² für Wasserstoff, R³ für Ethyl stehen und R¹ und B für jede einzelne Verbindung jeweils einer Zeile der Tabelle A entsprechen.

### Tabelle 22:

Verbindungen der allgemeinen Formel I-A, worin A für A2, R² für Methyl, R³ für Ethyl stehen und R¹ und B für jede einzelne Verbindung jeweils einer Zeile der Tabelle A entsprechen.

### Tabelle 23:

Verbindungen der allgemeinen Formel I-A, worin A für A2, R² für Cl, R³ für Ethyl stehen und R¹ und B für jede einzelne Verbindung jeweils einer Zeile der Tabelle A entsprechen.

### Tabelle 24:

Verbindungen der allgemeinen Formel I-A, worin A für A2, R² für F, R³ für Ethyl stehen und R¹ und B für jede einzelne Verbindung jeweils einer Zeile der Tabelle A entsprechen.

Ganz besonders bevorzugt sind die folgenden Thiazolcarbonsäureanilide der Formel I:
2-Methyl-4-trifluormethylthiazol-5-carbonsäure-N-(3',4',5'-trifluorbiphenyl-2-yl)-amid,
2-Methyl-4-trifluormethylthiazol-5-carbonsäure-N-(2',4',5'-trifluorbiphenyl-2-yl)-amid,
2,4-Dimethylthiazol-5-carbonsäure-N-(3',4',5'-trifluorbiphenyl-2-yl)-amid und
2,4-Dimethylthiazol-5-carbonsäure-N-(2',4',5'-trifluorbiphenyl-2-yl)-amid.

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und *Basidiomyceten.* Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria* Arten an Gemüse, Raps, Zuckerrüben und Obst und Reis, z.B. *A. solani* oder *A. alternata* an Kartoffeln und Tomaten,
- *Aphanomyces* Arten an Zuckerrüben und Gemüse,
- *Ascochyta* Arten an Getreide and Gemüse,
- *Bipolaris* und *Drechslera* Arten an Mais, Getreide, Reis und Rasen, z.B. *D. maydis* an Mais,
- *Blumeria graminis* (Echter Mehltau) an Getreide,
- *Botrytis cinerea* (Grauschimmel) an Erdbeeren, Gemüse, Blumen und Weinreben,
- *Bremia lactucae* an Salat,
- *Cercospora* Arten an Mais, Sojabohnen, Reis und Zuckerrüben,
- *Cochliobolus* Arten an Mais, Getreide, Reis, z.B. *Cochliobolus sativus* an Getreide, *Cochliobolus miyabeanus* an Reis,
- *Colletotricum* Arten an Sojabohnen und Baumwolle,
- *Drechslera* Arten, *Pyrenophora* Arten an Mais, Getreide, Reis und Rasen, z.B. *D. teres* an Gerste oder *D. tritici-repentis* an Weizen,
- *Esca* an Weinrebe, verursacht durch *Phaeoacremonium chlamydosporium, Ph. Aleophilum* und *Formitipora punctata (syn. Phellinus punctatus*)*,*
- *Exserohilum* Arten an Mais,
- *Erysiphe cichoracearum* und *Sphaerotheca fuliginea* an Gurkengewächsen,
- *Fusarium* und *Verticillium* Arten an verschiedenen Pflanzen, z.B. *F. graminearum* oder F. *culmorum* an Getreide oder *F*. *oxysporum* an einer Vielzahl von Pflanzen, z.B. Tomaten,
- *Gaeumanomyces graminis* an Getreide,
- *Gibberella* Arten an Getreide und Reis (z.B. *Gibberella fujikuroi* an Reis),
- *Grainstaining complex* an Reis,
- *Helminthosporium* Arten an Mais und Reis,
- *Michrodochium nivale* an Getreide,
- *Mycosphaerella* Arten an Getreide, Bananen und Erdnüssen, z.B. *M. graminicola* an Weizen oder *M*. *fijiesis* an Bananen,
- *Peronospora-Arten* an Kohl und Zwiebelgewächsen, z.B. *P. brassicae* an Kohl oder *P. destructor* an Zwiebel,
- *Phakopsara pachyrhizi* und *Phakopsara meibomiae* an Sojabohnen,
- *Phomopsis* Arten an Sojabohnen und Sonnenblumen,
- *Phytophthora infestans* an Kartoffeln und Tomaten,
- *Phytophthora* Arten an verschiedenen Pflanzen, z.B. *P. capsici* an Paprika,
- *Plasmopara viticola* an Weinreben,
- *Podosphaera leucotricha* an Apfel,
- *Pseudocercosporella herpotrichoides* an Getreide,
- *Pseudoperonospora* an verschiedenen Pflanzen, z.B. *P. cubensis* an Gurke oder *P. humili* an Hopfen,
- *Puccinia* Arten an verschiedenen Pflanzen, z.B. *P. triticina , P. striformins, P. hordei* oder *P. graminis* an Getreide oder *P. asparagi* an Spargel,
- *Pyricularia oryzae , Corticium sasakii, Sarocladium oryzae, S. attenuatum, Entyloma oryzae* an Reis,
- *Pyricularia grisea* an Rasen und Getreide,
- *Pythium spp.* an Rasen, Reis, Mais, Baumwolle, Raps, Sonnenblumen, Zuckerrüben, Gemüse und anderen Pflanzen, z.B. *P. ultiumum* an verschiedenen Pflanzen, *P. aphanidermatum* an Rasen,
- *Rhizoctonia* Arten an Baumwolle, Reis, Kartoffeln, Rasen, Mais, Raps, Zuckerrüben, Gemüse und an verschiedenen Pflanzen, z.B. *R. solani* an Rüben und verschiedenen Pflanzen,
- *Rhynchosporium secalis* an Gerste, Roggen und Triticale,
- *Sclerotinia* Arten an Raps und Sonnenblumen,
- *Septoria tritici* und *Stagonospora nodorum* an Weizen,
- *Erysiphe* (syn. *Uncinula*) *necator* an Weinrebe,
- *Setospaeria* Arten an Mais und Rasen,
- *Sphacelotheca reilinia* an Mais,
- *Thievaliopsis* Arten an Sojabohnen und Baumwolle,
- *Tilletia* Arten an Getreide,
- *Ustilago* Arten an Getreide, Mais und Zuckerrohr, z.B. *U. maydis* an Mais,
- *Venturia* Arten (Schorf) an Äpfeln und Birnen, z.B. *V. inaequalis* an Apfel.

Insbesondere eigenen sich die Verbindungen I zur Bekämpfung von Schadpilzen aus der Klasse der Peronosporomyceten (syn. Oomyceten) wie Peronospora-Arten, Phytophthera-Arten, Plasmopara viticola, Pseudoperonospora-Arten und Pythium-Arten.

Die Verbindungen I eignen sich außerdem zur Bekämpfung von Schadpilzen im Materialschutz (z.B. Holz, Papier, Dispersionen für den Anstrich, Fasern bzw. Gewebe) und im Vorratsschutz. Im Holzschutz finden insbesondere folgende Schadpilze Beachtung: Ascomyceten wie *Ophiostoma* spp., *Ceratocystis* spp., *Aureobasidium pullutans, Sclerophoma* spp., *Chaetomium* spp., *Humicola* spp., *Petriella spp., Trichurus* spp.; Basidiomyceten wie *Coniophora* spp., *Coriolus* spp., *Gloeophyllum* spp., *Lentinus* spp., *Pleurotus* spp., *Poria* spp., *Serpula* spp. und *Tyromyces* spp., Deuteromyceten wie *Aspergillus* spp., *Cladosporium* spp., *Penicillium* spp., *Trichoderma* spp., *Alternaria* spp., *Paecilomyces* spp. und Zygomyceten wie *Mucor* spp., darüber hinaus im Materialschutz folgende Hefepilze: *Candida* spp. und *Saccharomyces cerevisae.*

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen von 0,1 bis 95, vorzugsweise 0,5 bis 90 Gew.-% Wirkstoff.

Die Aufwandmengen liegen bei der Anwendung im Pflanzenschutz je nach Art des gewünschten Effektes bei 0,01 bis 2,0 kg Wirkstoff pro ha.

Bei der Saatgutbehandlung werden im allgemeinen Wirkstoffmengen von 1 bis 1000g/100kg, vorzugsweise 5 bis 100g/100kg, Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso^{®} Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (N-Methyl-pyrrolidon, N-Octylpyrrolidon), Acetate (Glykoldiacetat), Glykole, Dimethylfettsäureamide, Fettsäuren und Fettsäureester. Grundsätzlich können auch Lösungsmittelgemische verwendet werden.
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im allgemeinen von 0,01 bis 95 Gew.-%, vorzugsweise 0,1 bis 90 Gew.-%, mindestens eines Wirkstoffs I. Die Wirkstoffe werden dabei in einer Reinheit von 90% bis 100%, vorzugsweise 95% bis 100% (nach NMR-Spektrum) eingesetzt.

### Beispiele für Formulierungen sind: 1. Produkte zur Verdünnung in Wasser

### A Wasserlösliche Konzentrate (SL, LS)

10 Gew.-Teile einer efindungsgemäßen Verbindung I werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate (DC)

20 Gew.-Teile einer erfindungsgemäßen Verbindung I werden in 70 Gew.-Teilen Cyclohexanon unter Zusatz von 10 Gew.-Teilen eines Dispergiermittels, z.B. Polyvinylpyrrolidon, gelöst. Bei Verdünnung in Wasser ergibt sich eine Dispersion. Der Wirkstoffgehalt beträgt 20 Gew.-%.

### C Emulgierbare Konzentrate (EC)

15 Gew.-Teile einer erfindungsgemäßen Verbindung I werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen (EW, EO, ES)

25 Gew.-Teile einer efindungsgemäßen Verbindung I werden in 35 Gew.-Teilen Xylol unter Zusatz von Ca-Dodecylbenzolsulfonat und Ricinusölethoxylat (jeweils 5 Gew.-Teile) gelöst. Diese Mischung wird mittels einer Emulgiermaschine (Ultraturrax) in 30 Gew.-Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen (SC, OD, FS)

20 Gew.-Teile einer erfindungsgemäßen Verbindung I werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.

### F Wasserdispergierbare und wasserlösliche Granulate (WG, SG)

50 Gew.-Teile einer erfindungsgemäßen Verbindung I werden unter Zusatz von 50 Gew.-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.
G Wasserdispergierbare und wasserlösliche Pulver (WP, SP, SS, WS) 75 Gew.-Teile einer erfindungsgemäßen Verbindung I werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen (GF)

In einer Kugelmühle werden 20 Gew.-Teile einer erfindungsgemäßen Verbindung I, 10 Gew.-Teile Dispergiermittel, 1 Gew.-Teil Quellmittel ("gelling agent") und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### J Stäube (DP, DS)

5 Gew.Teile einer erfindungsgemäßen Verbindung I werden fein gemahlen und mit 95 Gew.-Teilen feinteiligem Kaolin innig vemischt. Man erhält dadurch ein Stäubemittel mit 5 Gew.-% Wirkstoffgehalt.

### K Granulate (GR, FG, GG, MG)

0,5 Gew.-Teile einer erfindungsgemäßen Verbindung I werden fein gemahlen und mit 99,5 Gew.-Teilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### L ULV- Lösungen (UL)

10 Gew.-Teile einer erfindungsgemäßen Verbindung I werden in 90 Gew.-Teilen eines organischen Lösungsmittels, z.B. Xylol, gelöst. Dadurch erhält man ein Produkt für die Direktapplikation mit 10 Gew.-% Wirkstoffgehalt.

Für die Saatgutbehandlung werden überlicherweise wasserlösliche Konzentrale (LS), Suspensionen (FS), Stäube (DS), wasserdispergierbare oder wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Die Wirkstoffe können als solche, in Form ihrer Formulierungen oder den daraus bereiteten Anwendungsformen, z.B. in Form von direkt versprühbaren Lösungen. Pulvern, Suspensionen oder Dispersionen, Emulsionen, Öldispersionen, Pasten, Stäubmitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe I gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie bei 0,0001 bis 10%, vorzugsweise 0,01 bis 1 %.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel werden üblicherweise zu den erfindungsgemäßen Mitteln im Gewichtsverhältnis 1:100 bis 100: 1, bevorzugt 1:10 bis 10:1, zugemischt.

Als Adjuvants in diesem Sinne kommen insbesondere in Frage: organisch modifizierte Polysiloxane, z.B. Break Thru, S. 240^{®}; Alkoholalkoxylate, z.B. Atplus 245^{®}, Atplus MBA 1303^{®}, Plurafac^{®} LF 300 und Lutensol^{®} ON 30; EO-PO-Blockpolymerisate, z.B. Pluronic^{®} RPE 2035 und Genapol^{®} B; Alkoholethxylate, z.B. Lutensol^{®} XP80; und Natriumdioctylsulfosuccinat, z.B. Leophen^{®} RA.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren wie Prohexadion Ca, Fungiziden oder auch mit Düngemitteln. Beim Vermischen der Verbindungen I bzw. der sie enthaltenden Mittel mit einem oder mehreren weiteren Wirkstoffen, insbesondere Fungiziden, kann beispielsweise in vielen Fällen das Wirkungsspektrum verbreitert werden oder Resistenzentwicklungen vorgebeugt weiden. In vielen Fällen erhält man dabei synergistische Effekte.

Die folgende Liste von Fungiziden, mit denen die erfindungsgemäßen Verbindungen gemeinsam angewendet werden können, soll die Kombinationsmöglichkeiten erläutem, nicht aber einschränken:

### Strobilurine

Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethyl ester, 2-(ortho-(2,5-Dimethylphenyl-oxymethylen)phenyl)-3-methoxy-acrylsäuremethylester;

### Carbonsäureamide

- Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3,4-Dichlor-isothiazol-5-carbonsäure-(2-cyano-phenyl)-amid;
- Carbonsäuremorpholide: Dimethomorph, Flumorph;
- Benzoesäureamide: Flumetover, Fluopicolid (Picobenzamid), Zoxamid;
- Sonstige Carbonsäureamide: Carpropamid, Diclocymet, Mandipropamid, N-(2-(4-[3-(4-Chlor-phenyl)-prop-2-inyloxy)-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;

### Azole

- Triazole: Bitertanol, Bromuconazol, Cyproconazol, Difenoconazol, Diniconazol, Enilconazol, Epoxiconazol, Fenbuconazol, Flusilazol, Fluquinconazol, Flutriafol, Hexaconazol, Imibenconazol, Ipconazol, Metconazol, Myclobutanil, Penconazol, Propiconazol, Prothioconazol, Simeconazol, Tebuconazol, Tetraconazol, Triadimenol, Triadimefon, Triticonazol;
- Imidazole: Cyazofamid, Imazalil, Pefurazoat, Prochloraz, Triflumizol;
- Benzimidazole: Benomyl, Carbendazim, Fuberidazol, Thiabendazol;
- Sonstige: Ethaboxam, Etridiazol, Hymexazol;

### Stickstoffhaltige Heterocyclylverbindungen

- Pyridine: Fluazinam, Pyrifenox, 3-[5-(4-Chlor-phenyl)-2,3-dimethyl-isoxazolidin-3-yl]-pyridin;
- Pyrimidine: Bupirimate, Cyprodinil, Ferimzone, Fenarimol, Mepanipyrim, Nuarimol, Pyrimethanil;
- Piperazine: Triforin;
- Pyrrole: Fludioxonil, Fenpiclonil;
- Morpholine: Aldimorph, Dodemorph, Fenpropimorph, Tridemorph;
- Dicarboximide: Iprodion, Procymidon, Vinclozolin;
- sonstige: Acibenzolar-S-methyl, Anilazin, Captan, Captafol, Dazomet, Diclomezin Fenoxanil, Folpet, Fenpropidin, Famoxadon, Fenamidon, Octhilinon, Probenazol, Proquinazid, Pyroquilon, Quinoxyfen, Tricyclazol, 5-Chlor-7-(4-methyl-piperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluoro-2-methyl-indol-1-sulfonyl)-[1,2,4]triazol-1-sulfonsäuredimethylamid;

### Carbamate und Dithiocarbamate

- Dithiocarbamate: Ferbam, Mancozeb, Maneb, Metiram, Metam, Propineb, Thiram, Zineb, Ziram;
- Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluorphenyl)ester,

### Sonstige Fungizide

- Guanidine: Dodine, Iminoctadine, Guazatine;
- Antibiotika: Kasugamycin, Polyoxine, Streptomycin, Validamycin A;
- Organometallverbindungen: Fentin Salze;
- Schwefelhaltige Heterocyclylverbindungen: Isoprothiolane, Dithianon;
- Organophosphorverbindungen: Edifenphos, Fosetyl, Fosetyl-aluminium, Iprobenfos, Pyrazophos, Tolclofos-methyl, Phosphorige Säure und ihre Salze;
- Organochlorverbindungen: Thiophanat Methyl, Chlorthalonil, Dichlofluanid, Tolylfluanid, Flusulfamid, Phthalid, Hexachlorbenzol, Pencycuron, Quintozen;
- Nitrophenylderivate: Binapacryl, Dinocap, Dinobuton;
- Anorganische Wirkstoffe: Bordeaux Brühe, Kupferacetat, Kupferhydroxid, Kupferoxychlorid, basisches Kupfersulfat, Schwefel;
- Sonstige: Spiroxamin, Cyflufenamid, Cymoxanil, Metrafenon.

### Synthesebeispiele

### 2-Methyl-4-trifluormethylthiazol-5-carbonsäure (3',4',5'-trifluorbiphenyl-2-yl)amid (Bsp. I.1):

Zu einer Lösung von 0,40 g 2-Methyl-4-trifluormethylthiazol-5-carbonsäure und 0,38 g Triethylamin in 30 ml Dichlormethan wurden bei Raumtemperatur 0,42 g 3',4',5'-. Trifluorbiphenyl-2-ylamin und 0,72 g Bis-(2-oxo-3-oxazolidinyl)phosphorylchlorid gegeben. Die Mischung wurde 16 Stunden bei Raumtemperatur gerührt. Danach wurde sukzessive zweimal mit verdünnter Salzsäure, zweimal mit wässriger Natriumhydrogencarbonat-Lösung und einmal mit Wasser gewaschen. Die organische Phase wurde getrocknet und eingeengt. Das Rohprodukt wurde durch Säulenchromatographie mit Cyclohexan/Methyl-tert-butylether 1:2 an Kieselgel gereinigt. Man erhielt dadurch 0,61 g des gewünschten Produktes in Form hellbrauner Kristalle vom Smp. 148-152°C.

Nach den hier angegebenen Vorschriften wurden die in der nachfolgenden Tabelle 25 angegebenen Verbindungen der allgemeinen Formel I hergestellt, in der A für A1 steht.

**Tabelle 25**

| **Beispiel** | **R¹** | **R²** | **R³** | **X** | **Y** | **p** | **w** | **Charakterisierung (Smp. oder** **¹H-NMR)** |
|---|---|---|---|---|---|---|---|---|
| I.1 | CF₃ | CH₃ | H | 3,4,5-F₃ | - | 0 | O | 148-152°C |
| I.2 | CF₃ | CH₃ | H | 2,4,5-F₃ | - | 0 | O | 112-116°C |
| I.3 | CH₃ | CH₃ | H | 3,4,5-F₃ | - | 0 | O | 123-128°C |
| I.4 | CH₃ | CH₃ | H | 2,4,5-F₃ | - | 0 | O | 150-154°C |

### Beispiele für die Wirkung gegen Schadpilze

Die fungizide Wirkung der Verbindungen der Formel I ließ sich durch die folgenden Versuche zeigen:

Die Wirkstoffe wurden als eine Stammlösung aufbereitet mit 25 mg Wirkstoff, welcher mit einem Gemisch aus Aceton und/oder Dimethylsulfoxid und dem Emulgator Uniperol® EL (Netzmittel mit Emulgier und Dispergierwirkung auf der Basis ethoxylierter Alkylphenole) in Volumen-Verhältnis Lösungsmittel / Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel/Emulgator/Wasser-Gemisch zu der unten angegebenen Wirkstoffkonzentration verdünnt.

### Anwendungsbeispiel 1 - Kurative Wirksamkeit gegen Weizenbraunrost verursacht durch Puccinia recondita

Blätter von in Töpfen gewachsenen Weizensämlingen der Sorte "Kanzler" wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22°C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropfnässe besprüht. Die Suspension oder Emulsion wurde wie oben beschrieben hergestellt Nach dem Antrocknen des Spritzbelages wurden die Versuchspflanzen im Gewächshaus bei Temperaturen zwischen 20 und 22°C und 65 bis 70 % relativer Luftfeuchte für 7 Tage kultiviert. Dann wurde das Ausmaß der Rostpilzentwicklung auf den Blättern ermittelt.

In diesem Test zeigten die mit 250 mg/l der Verbindungen I.1, I.2, I.3 und I.4 aus Tabelle 25 behandelten Pflanzen maximal 1% Befall, während die unbehandelten Pflanzen zu 90% befallen waren.

### Anwendungsbeispiel 2 - Wirksamkeit gegen die Dürrfleckenkrankheit der Tomate verursacht durch Alternaria solani

Blätter von Topfpflanzen der Sorte "Goldene Königin" wurden mit einer wässrigen Suspension in der unten angegebenen Wirkstoffkonzentration bis zur Tropfnässe besprüht. Am folgenden Tag wurden die Blätter mit einer wässrigen Sporenaufschwemmung von *Alternaria solani* in 2% Biomalzlösung mit einer Dichte von 0.12 x 10⁶ Sporen/ml infiziert. Anschließend wurden die Pflanzen in einer wasserdampf-gestättigten Kammer bei Temperaturen zwischen 20 und 22°C aufgestellt. Nach 5 Tagen hatte sich die Krankheit auf den unbehandelten, jedoch infizierten Kontrollpflanzen so stark entwickelt, dass der Befall visuell in % ermittelt werden konnte.

In diesem Test zeigten die mit 4 ppm der Verbindung I.1 aus der Tabelle 25 behandelten Pflanzen 7% Befall, während die unbehandelten Pflanzen zu 90 % befallen waren.

Die mit 4 ppm der aus WO 2003/066609 bekannten Vergleichsverbindung 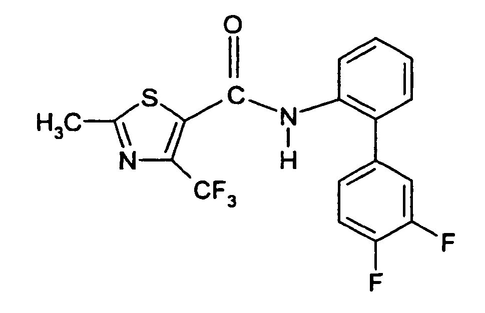 behandelten Pflanzen zeigten 20% Befall.

## Patentansprüche

1. Thiazolcarbonsäureanilide der Formel I 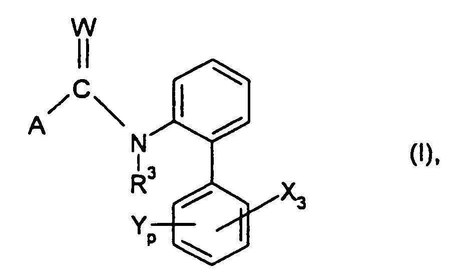 in der die Variablen folgende Bedeutungen haben:
A 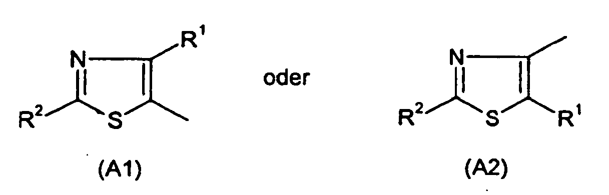
X Halogen;
Y Cyano, Nitro, C₁-C₄-Alkyl, C₁-C₄-Halogenalkyl, Methoxy oder Methylthio;
p 0 oder 1;
R¹ Wasserstoff, Halogen, C₁-C₄-Alkyl oder C₁-C₄-Halogenalkyl;
R² Wasserstoff, Methyl oder Halogen;
R³ Wasserstoff, Methyl oder Ethyl;
W Sauerstoff oder Schwefel.

2. Thiazolcarbonsäureanilide der Formel I nach Anspruch 1, in der die Variablen folgende Bedeutungen haben:
X F oder Chlor;
Y C₁-C₄-Alkyl, C₁-C₄-Halogenalkyl oder Methoxy;
p 0 oder 1;
R¹ Wasserstoff, Halogen, C₁-C₄-Alkyl oder C₁-C₄-Halogenalkyl;
R² Wasserstoff, Methyl oder Halogen;
R³ Wasserstoff oder Methyl;
W Sauerstoff.

3. Thiazolcarbonsäureanilide der Formel I nach einem der Ansprüche 1 oder 2, in der die Variablen folgende Bedeutungen haben:
X F oder Chlor;
Y Methyl, Difluormethyl, Trifluormethyl oder Methoxy;
p 0 oder 1;
R¹ Wasserstoff, F, Cl, Methyl, Fluormethyl, Difluormethyl, Chlorfluormethyl, Chlordifluormethyl, Dichlorfluormethyl oder Trifluormethyl;
R² Wasserstoff, F, Cl oder Methyl;
R³ Wasserstoff oder Methyl;
W Sauerstoff.

4. Thiazolcarbonsäureanilide der Formel I nach einem der Ansprüche 1 bis 3, in der die Variablen folgende Bedeutungen haben:
X F oder Chlor;
p Null;
R¹ Wasserstoff, F, Cl, Methyl, Fluormethyl, Difluormethyl, Chlorfluormethyl, Chlordifluormethyl, Dichlorfluormethyl oder Trifluormethyl;
R² Wasserstoff, F, Cl oder Methyl;
R3 Wasserstoff;
W Sauerstoff.

5. Thiazolcarbonsäureanilide der Formel I nach einem der Ansprüche 1 bis 4, in der die Variablen folgende Bedeutungen haben:
X F oder Chlor;
p Null;
R¹ Wasserstoff, F, Cl, Methyl, Fluormethyl, Difluormethyl, Chlorfluormethyl, Chlordifluormethyl, Dichlorfluormethyl oder Trifluormethyl;
R² Wasserstoff, Cl oder Methyl;
R³ Wasserstoff;
W Sauerstoff.

6. Thiazolcarbonsäureanilide der Formel I nach einem der Ansprüche 1 bis 5, in der der Substituent A die Bedeutung A1 hat.

7. Thiazolcarbonsäureanilide der Formel I nach Anspruch 1, ausgewählt aus der Gruppe bestehend aus 2-Methyl-4-trifluormethylthiazol-5-carbonsäure-N-(3',4',5'-trifluorbiphenyl-2-yl)-amid, 2-Methyl-4-trifluormethylthiazol-5-carbonsäure-N-(2',4',5'-trifluorbiphenyl-2-yl)-amid, 2,4-Dimethylthiazol-5-carbonsäure-N-(3',4',5'-trifluorbiphenyl-2-yl)-amid und 2,4-Dimethylthiazol-5-carbonsäure-N-(2',4',5'-trifluorbiphenyl-2-yl)-amid.

8. Mittel zur Bekämpfung von Schadpilzen, enthaltend eine fungizide Menge mindestens einer Verbindung der Formel I gemäß einem der Ansprüche 1 bis 7 und mindestens einem inerten Zusatzstoff.

9. Verfahren zur Bekämpfung von pflanzenpathogenen Schadpilzen, **dadurch gekennzeichnet, dass** man die Schadpilze, ihren Lebensraum und/oder die vor Pilzbefall zu schützenden Materialien, Pflanzen, den Boden oder Saatgüter mit einer fungizid wirksamen Menge mindestens einer Verbindung der Formel I gemäß einem der Ansprüche 1 bis 7 behandelt.

10. Verwendung der Verbindungen I gemäß einem der Ansprüche 1 bis 7 zur Bekämpfung von pflanzenpathogenen Schadpilzen.

11. Saatgut, enthaltend mindestens eine Verbindung der Formel I gemäß einem der Ansprüche 1 bis 7 in einer Menge von 1 bis 1000g/100kg Saatgut.

12. 3',4',5'-Trifluorbiphenyl-2-ylamin.

## Claims

1. A thiazolecarboxanilide of the formula I 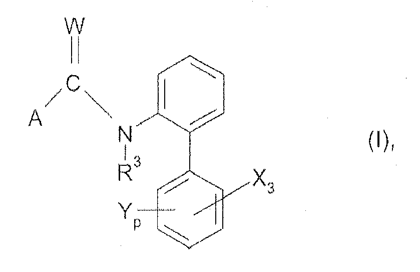 in which the variables are as defined below:
A is 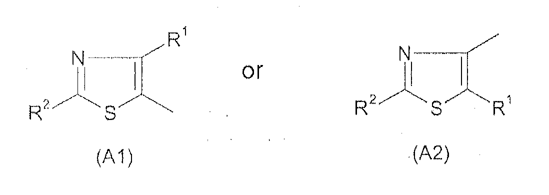
X is halogen;
Y is cyano, nitro, C₁-C₄-alkyl, C₁-C₄-haloalkyl, methoxy or methylthio;
p is 0 or 1;
R¹ is hydrogen, halogen, C₁-C₄-alkyl or C₁-C₄-haloalkyl;
R² is hydrogen, methyl or halogen;
R³ is hydrogen, methyl or ethyl;
W is oxygen or sulfur.

2. The thiazolecarboxanilide of the formula I according to claim 1 in which the variables are as defined below:
X is F or chlorine;
Y is C₁-C₄-alkyl, C₁-C₄-haloalkyl or methoxy;
p is 0, 1;
R¹ is hydrogen, halogen, C₁-C₄-alkyl or C₁-C₄-haloalkyl;
R² is hydrogen, methyl or halogen;
R³ is hydrogen or methyl;
W is oxygen.

3. The thiazolecarboxanilide of the formula I according to claim 1 or 2 in which the variables are as defined below:
X is F or chlorine;
Y is methyl, difluoromethyl, trifluoromethyl or methoxy;
p is 0 or 1;
R¹ is hydrogen, F, Cl, methyl, fluoromethyl, difluoromethyl, chlorofluoromethyl, chlorodifluoromethyl, dichlorofluoromethyl or trifluoromethyl;
R² is hydrogen, F, Cl or methyl;
R³ is hydrogen or methyl;
W is oxygen.

4. The thiazolecarboxanilide of the formula I according to any of claims 1 to 3 in which the variables are as defined below:
X is F or chlorine;
p is zero;
R¹ is hydrogen, F, Cl, methyl, fluoromethyl, difluoromethyl, chlorofluoromethyl, chlorodifluoromethyl, dichlorofluoromethyl or trifluoromethyl;
R² is hydrogen, F, Cl or methyl;
R³ is hydrogen;
W is oxygen.

5. The thiazolecarboxanilide of the formula according to any of claims 1 to 4 in which the variables are as defined below:
X is F or chlorine;
p is zero;
R¹ is hydrogen, F, Cl, methyl, fluoromethyl, difluoromethyl, chlorofluoromethyl, chlorodifluoromethyl, dichlorofluoromethyl or trifluoromethyl;
R² is hydrogen, Cl or methyl;
R³ is hydrogen;
W is oxygen.

6. The thiazolecarboxanilide of the formula I according to any of claims 1 to 5 in which the substituent A has the meaning A1.

7. The thiazolecarboxanilide of the formula I according to claim 1, selected from the group consisting of N-(3',4',5'-trifluorobiphenyl-2-yl)-2-methyl-4-trifluoromethylthiazole-5-carboxamide, N-(2',4',5'-trifluorobiphenyl-2-yl)-2-methyl-4-trifluoromethylthiazole-5-carboxamide, N-(3',4',5'-trifluorobiphenyl-2-yl)-2,4-dimethylthiazole-5-carboxamide and N-(2',4',5'-trifluorobiphenyl-2-yl)-2,4-dimethylthiazole-5-carboxamide.

8. A composition for controlling harmful fungi, which comprises a fungicidal amount of at least one compound of the formula I according to any of claims 1 to 7 and at least one inert additive.

9. A method for controlling phytopathogenic harmful fungi which comprises treating the harmful fungi, their habitat and/or the materials, plants, the soil or seed to be protected against fungal attack with a fungicidally effective amount of at least one compound of the formula I according to any of claims 1 to 7.

10. The use of a compound I according to any of claims 1 to 7 for controlling phytopathogenic harmful fungi.

11. A seed, comprising at least one compound of the formula I according to any of claims 1 to 7 in an amount of from 1 to 1000 g/100 kg of seed.

12. 3', 4', 5'-Trifluorobiphenyl-2-ylamine.

## Revendications

1. Anilides d'acide thioazolocarboxylique de formule I 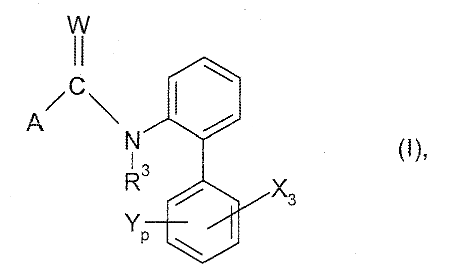 dans laquelle les variables ont les significations suivantes :
A 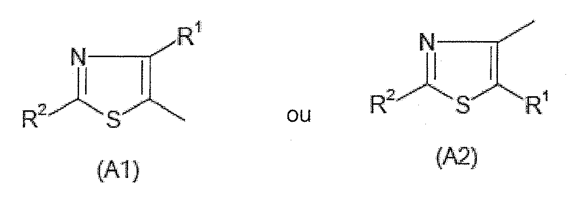
X représente un halogène;
Y représente un groupe cyano, nitro, alkyle en C₁ à C₄, halogénoalkyle en C₁ à C₄, méthoxy ou méthylthio;
p représente 0 ou 1;
R¹ représente l'hydrogène, un halogène, un groupe alkyle en C₁ à C₄ ou halogénoalkyle en C₁ à C₄;
R² représente l'hydrogène, un groupe méthyle ou un halogène;
R³ représente l'hydrogène, un groupe méthyle ou éthyle et
W représente l'oxygène ou le soufre.

2. Anilides d'acide thioazolocarboxylique de formule I selon la revendication 1, dans laquelle les variables ont les significations suivantes :
X représente F ou le chlore;
Y représente un groupe alkyle en C₁ à C₄, halogénoalkyle en C₁ à C₄ ou méthoxy;
p représente 0 ou 1;
R¹ représente l'hydrogène, un halogène, un groupe alkyle en C₁ à C₄ ou halogénoalkyle en C₁ à C₄;
R² représente l'hydrogène, un groupe méthyle ou un halogène;
R³ représente l'hydrogène ou un groupe méthyle et
W représente l'oxygène.

3. Anilides d'acide thioazolocarboxylique de formule I selon l'une des revendications 1 ou 2, dans laquelle les variables ont les significations suivantes :
X représente F ou le chlore;
Y représente un groupe méthyle, difluorométhyle, trifluorométhyle ou méthoxy;
p représente 0 ou 1;
R¹ représente l'hydrogène, F, CI, un groupe méthyle, fluorométhyle, difluorométhyle, chlorofluorométhyle, chlorodifluorométhyle, dichlorofluorométhyle ou trifluorométhyle;
R² représente l'hydrogène, F, Cl ou un groupe méthyle;
R³ représente l'hydrogène ou un groupe méthyle et
W représente l'oxygène.

4. Anilides d'acide thioazolocarboxylique de formule I selon l'une des revendications 1 à 3, dans laquelle les variables ont les significations suivantes :
X représente F ou le chlore;
p représente 0;
R¹ représente l'hydrogène, F, CI, un groupe méthyle, fluorométhyle, difluorométhyle, chlorofluorométhyle, chlorodifluorométhyle, dichlorofluorométhyle ou trifluorométhyle;
R² représente l'hydrogène, F, Cl ou un groupe méthyle;
R³ représente l'hydrogène et
W représente l'oxygène.

5. Anilides d'acide thioazolocarboxylique de formule I selon l'une des revendications 1 à 4, dans laquelle les variables ont les significations suivantes :
X représente F ou le chlore;
p représente 0;
R¹ représente l'hydrogène, F, CI, un groupe méthyle, fluorométhyle, difluorométhyle, chlorofluorométhyle, chlorodifluorométhyle, dichlorofluorométhyle ou trifluorométhyle;
R² représente l'hydrogène, Cl ou un groupe méthyle;
R³ représente l'hydrogène et
W représente l'oxygène.

6. Anilides d'acide thioazolocarboxylique de formule I selon l'une des revendications 1 à 5, dans laquelle le substituant A représente A1.

7. Anilides d'acide thioazolocarboxylique de formulé I selon la revendication 1, sélectionnés dans l'ensemble constitué du N-(3',4',5'-trifluorbiphényl-2-yl)-amide de l'acide 2-méthyl-4-trifluorméthylthiazol-5-carboxylique, du N-(2',4',5'-trifluorbiphényl-2-yl)-amide de l'acide 2-méthyl-4-trifluorméthylthiazol-5-carboxylique, du N-(3',4',5'-trifluorbiphényl-2-yl)-amide de l'acide 2,4-diméthylthiazol-5-carboxylique et du N-(2',4',5'-trifluorbiphényl-2-yl)-amide de l'acide 2,4-diméthylthiazol-5-carboxylique.

8. Agent de lutte contre les champignons nuisibles, qui contient une quantité fongicide d'au moins un composé de formule 1 selon l'une des revendications 1 à 7 et au moins un additif inerte.

9. Procédé de lutte contre les champignons nuisibles phytopathogènes, **caractérisé en ce que** l'on traite les champignons nuisibles, leur espace vital et/ou les matériaux à protéger de l'attaque par les champignons, des plantes, le sol ou des produits de semences avec une quantité efficacement fongicide d'au moins un composé de formule I selon l'une des revendications 1 à 7.

10. Utilisation des composés I selon l'une des revendications 1 à 7 dans la lutte contre les champignons nuisibles phytopathogènes.

11. Produit de semence qui contient au moins un composé de formule I selon l'une des revendications 1 à 7 en quantité de 1 à 1 000 g/100 kg de produit de semence.

12. 3', 4', 5' -Trifluorbiphényl-2-ylamine.
